# EUROPÄISCHE PATENTSCHRIFT

(11) **EP 0 482 445 B1**
(45) Veröffentlichungstag und Bekanntmachung des Hinweises auf die Patenterteilung: **03.08.1994**
(21) Anmeldenummer: 91117344.1
(22) Anmeldetag: 11.10.1991
(51) Int. Cl.: C07C 29/17, C07C 31/20, B01J 23/74

(54) **Verfahren zur Hydrierung von Acetylenalkoholen**
Method for the hydrogenation of acetylenealcohols
Procédé pour l'hydrogénation d'alcools acéthyléniques

(30) Priorität: 19.10.1990 US 599877
(43) Veröffentlichungstag der Anmeldung: 29.04.1992
(73) Patentinhaber: BASF Aktiengesellschaft, 67063 Ludwigshafen (DE)
(72) Erfinder: Schossig, Juergen, Dr., W-6701 Fussgoenheim (DE); Koppenhoefer, Gerhard, Dr., W-6725 Roemerberg (DE); Irgang, Matthias, Dr., W-6900 Heidelberg (DE); Schnur, Rudolf, Baton Rouge, LA 70815 (US); Roley, George, Baton Rouge, LA 70816 (US)

(56) Entgegenhaltungen:
- EP-A- 0 394 841
- US-A- 2 222 302
- World Patent Index database, Derwent Publ. Ltd. London, GB. Accession Nr. 73-62896U, Derwent Wk.

## Beschreibung

Diese Erfindung betrifft ein Verfahren zur Herstellung gesättigter Alkohole durch katalytische Hydrierung von Acetylenalkoholen.

Für die Hydrierung aliphatischer ungesättigter Verbindungen sind zahlreiche Katalysatoren beschrieben worden.

Aus der DE-A-25 36 273 sind trägerfreie Nickelkatalysatoren für die technische Butindiolhydrierung bekannt, welche die Oxide von Nickel, Kupfer, Molybdän und Mangan enthalten. Es werden unter verschärften Reaktionsbedingungen zwar gute Resultate erhalten, welche jedoch mit einer erhöhten Bildung an unerwünschtem Butanol erkauft werden muß.

Aus der US-A-3 449 445 sind Katalysatoren bekannt, welche Nickel, Kupfer und Mangan auf Siliziumdioxid enthalten. Bei diesen treten im großtechnischen Dauerbetrieb in Hydrier-und Aufarbeitungsanlagen Ablagerungen von Siliziumdioxid in den Wärmetauschern und Rohrleitungen auf, welche sich nur durch sehr aufwendige Reinigungsoperationen entfernen lassen.

Aus der EP-A-18 569 sind Hydrierkatalysatoren bekannt, die die Oxide der Metalle Nickel, Kupfer, Molybdän und Mangan enthalten, die besonders vorteilhafte Ergebnisse bei der Hydrierung von But-2-in-1,4-diol zu Butan-1,4-diol erzielen, wenn man bei der Herstellung des Katalysators ein Aluminium- oder Eisensalz vor der Ausfällung der Metallsalze zugibt und die Masse anschließend durch Filtrieren, Waschen, Trocknen und Tempern behandelt. Beim Einsatz dieses Katalysators im großtechnischen Dauerbetrieb findet jedoch eine langsame, aber kontinuierliche Auflösung des Katalysators statt.

Es bestand deshalb die Aufgabe, ein neues und verbessertes Verfahren zur Hydrierung von Acetylenalkoholen zu entwickeln und den zuvor genannten Nachteilen abzuhelfen.

Demgemäß wurde ein neues und verbessertes Verfahren zur Herstellung gesättigter Alkohole durch katalytische Hydrierung von Acetylenalkoholen bei Temperaturen von 50 bis 200°C und Drücken von 30 bis 320 bar gefunden, welches dadurch gekennzeichnet ist, daß man einen Katalysator verwendet, der einen Gehalt von 20 bis 85 Gew.-% Nickeloxid, 0 bis 30 Gew.-% Kupferoxid, 1 bis 30 Gew.-% Zirkoniumdioxid, 1 bis 30 Gew.-% Siliziumdioxid und 1 bis 30 Gew.-% Aluminiumoxid aufweist, wobei sich die Anteile jeweils auf den oxidischen, nicht reduzierten Katalysator beziehen.

Als Katalysatoren eignen sich solche, die 20 bis 85 Gew.-% NiO, bevorzugt 30 bis 85 Gew.-% NiO, 0 bis 30 Gew.-% CuO, bevorzugt 0 bis 20 Gew.-%, besonders bevorzugt 0 bis 10 Gew.-% CuO, oder im wesentlichen Kupfer-frei, 1 bis 30 Gew.-% ZrO₂, bevorzugt 1 bis 20 Gew.-% ZrO₂, 1 bis 30 Gew.-% Al₂O₃, bevorzugt 1 bis 20 Gew.-% Al₂O₃, 1 bis 30 Gew.-% SiO₂, bevorzugt 1 bis 20 Gew.-% SiO₂, enthalten. Gegebenenfalls können die Katalysatoren noch weitere Oxide enthalten wie 0 bis 5 Gew.-% MoO₃ und 0 bis 5 Gew.-% MnO₂, wobei sich die Anteile jeweils auf den oxidischen, nicht reduzierten Katalysator beziehen.

Zur Herstellung der gesättigten, insbesondere zweiwertigen Alkohole sind grundsätzlich alle Acetylenalkohole geeignet, die nach üblichen Verfahren erhalten werden können, wie But-2-in-1,4-diol und Propargylalkohol.

Besonders gut ist das neue Verfahren für die Hydrierung von But-2-in-1,4-diol zu Butan-1,4-diol geeignet. Bei dieser Hydrierung wird eine wesentliche Steigerung des Durchsatzes bei deutlich verlängerter Katalysatorstandzeit erzielt.

Die für das erfindungsgemäße Verfahren verwendeten Katalysatoren stellt man zum Beispiel dadurch her, daß man die Salze der Metalle Nickel, Kupfer, Zirkonium, Aluminium, Silizium und gegebenenfalls Molybdän und Mangan auf an sich übliche Weise aus wässriger Lösung bei einer Temperatur von 30 bis 90°C und einem pH-Wert von 5 bis 9 ausfällt, die Suspension filtriert und den Filterkuchen trocknet und bei einer Temperatur von 300 bis 700°C tempert, wobei man das Molybdän als Ammoniummolybdat vor dem Trocknen zugibt. Dabei nimmt man die Fällung so vor, daß man eine wässrige Lösung von Salzen wie den Nitraten, Sulfaten oder Acetaten der Metalle Nickel, Kupfer, Zirkonium, Aluminium, Silizium und gegebenenfalls Mangan mit einer wässrigen Lösung von Alkalicarbonat vermischt. Die Menge der Metallsalze wird dabei so bemessen, daß die Katalysatormasse nach dem Tempern die angegebene Zusammensetzung aufweist.

Man kann die wasserlöslichen Salze von Zirkonium, Aluminium, Silizium zum Beispiel teilweise oder auch vollständig durch festes oder vorgefälltes ZrO₂, Al₂O₃, SiO₂ oder Alumosilikat ersetzen, welche man der wässrigen Metallsalzlösung vor der Fällung zugibt, im Rührgefäß vorlegt, im Rahmen einer Vorfällung erzeugt oder nach der Fällung zugibt.

Bei der Herstellung des Katalysators verfährt man im einzelnen zum Beispiel so, daß man die wässrige Lösung von Nickel, Kupfer, Zirkoniumsalzen gleichzeitig unter Rühren mit einer wässrigen Alkalicarbonatlösung vermischt, wobei die Metalle in Form eines Gemisches von Metallhydroxiden und Metallcarbonaten auf in einer Vorfällung hergestelltes Al₂O₃, SiO₂ ausfallen. Der Metallsalzgehalt beträgt zweckmäßigerweise 30 bis 40 Gew.-%. Die wässrige Alkalicarbonatlösung ist zum Beispiel 10 bis 20, vorzugsweise 15 bis 20 gew.-%ig. Man fällt bei 20 bis 90, vorzugsweise bei 35 bis 80°C und einem pH-Wert von 5 bis 9, vorzugsweise 6 bis 8.

Die erhaltene Suspension wird filtriert und so lange mit Wasser gewaschen, bis keine Anionen im Waschwasser nachgewiesen werden können. Dann wird zum Beispiel bei einer Temperatur von 120 bis 200°C im Trockenschrank oder einem Sprühtrockner getrocknet. Das Molybdän wird vorzugsweise als Ammoniumheptamolybdat dem feuchten Filterkuchen zugegeben. Der getrocknete Filterkuchen wird bei einer Temperatur von 350 bis 700°C, vorzugsweise 400 bis 600°C, getempert.

Zweckmäßigerweise wird die so erhaltene Katalysatormasse vor dem Einsatz auf übliche Weise tablettiert oder extrudiert. Zum Beispiel verpreßt man die Katalysatormasse mit einem Tablettierhilfsmittel, wie Graphit, auf einer Tablettenpresse. Die Tabletten mit der Dimension 5,0 x 3,0 mm haben ein Rüttelgewicht von 1000 bis 1500 g/l, eine Porosität (durch Wasseraufnahme bestimmt) von 0,2 auf 0,6 cm³/g und eine Härte von 2000 bis 5000 N/cm2.

Man kann aus der Katalysatormasse auch Extrudate herstellen, indem man sie mit Wasser und gegebenenfalls einer Säure, vorzugsweise Ameisensäure oder Salpetersäure, verknetet, extrudiert, trocknet und bei 500°C tempert. Im allgemeinen werden hierbei leichtere Katalysatoren erhalten.

Die so erhältlichen Katalysatoren werden zweckmäßig vor dem Einsatz zur Hydrierung von Acetylenalkoholen einer an sich üblichen Vorbehandlung unterworfen. Beispielsweise behandelt man den Katalysator zu diesem Zweck etwa 20 bis 40 Stunden bei Temperaturen von 150 bis 500°C, vorzugsweise 250 bis 500°C bei Wasserstoffdrücken von 1 bis 300 bar, vorzugsweise 100 bis 150 bar. Der Hydrierkatalysator weist in seiner reduzierten Form einen Reduktionsgrad von mindestens 50 % bezogen auf die Hydriermetalle auf. Zur gefahrlosen Handhabung der Katalysatoren an der Luft, erfolgt nach der Reduktion zweckmäßigerweise eine oberflächliche Passivierung durch Behandlung mit einem N₂/O₂-Gemisch bei Temperaturen von 10 bis 80°C, vorzugsweise von 40 bis 60°C.

Die Hydrierung der genannten ungesättigten Verbindungen wird bei den angegebenen Temperaturen und Drücken zum Beispiel in einem Reaktor durchgeführt, welcher den Katalysator in Form eines Festbettes enthält.

Wie aus den folgenden Beispielen hervorgeht, wurden hierbei überraschenderweise erheblich höhere Raum-Zeit-Ausbeuten und längere Katalysatorstandzeiten als bei den bekannten Verfahren erzielt.

### Beispiele

Die in den Beispielen genannten Prozente sind Gewichtsprozente.

### Herstellung der Katalysatoren

### Beispiel 1

Lösung 1: 22,5 g Al₂O₃ als Aluminiumsulfat wurden unter Zugabe von 24 ml konzentrierter Schwefelsäure in 187 ml Wasser gelöst. Lösung 2: 250 g Wasserglaslösung (27 % SiO₂) wurden in 864 g Wasser gelöst. Lösung 3 wurde durch Vereinigung von Lösung 1 und 2 hergestellt. Der pH-Wert dieser Lösung betrug 1,5.

In einem 10 l Rührgefäß wurden 1200 ml Wasser vorgelegt und Lösung 3 gleichzeitig mit insgesamt 140 g NH₄OH conz. bei einer Temperatur von 40°C gefällt. Die NH₄OH-Zugabe erfolgte dabei so, daß im Verlaufe der Fällung ein pH-Wert von 7,5 aufrecht erhalten wurde. Anschließend wurde über einen Zeitraum von 5 Minuten nachgerührt. Nun wurden 1466 g einer wässrigen Nickelnitrat-(337,5 g NiO)-Zirkonacetatlösung (22,5 g ZrO₂) über einen Zeitraum von 45 Minuten parallel mit insgesamt 3000 g Sodalösung (20%ig) bei einem pH-Wert von 6,0 und einer Temperatur von 40°C hinzugefällt. Nach Beendigung der Fällung wurde über einen Zeitraum von 60 Minuten unter kräftigem Rühren Luft eingeleitet.

Die erhaltene Suspension wurde filtriert und der Filterkuchen mit vollentsalztem Wasser gewaschen bis die elektrische Leitfähigkeit des Filtrates ca. 20 µs betrug. Danach wurde der Filterkuchen bei einer Temperatur von 150°C getrocknet und anschließend über einen Zeitraum von 4 Stunden bei einer Temperatur von 500°C getempert.

Der so erhaltene Katalysator hatte die Zusammensetzung: 75 % NiO, 15 % SiO₂, 5 % Al₂O₃, 5 % ZrO₂. Das Katalysatorpulver wurde mit 3 % Graphit vermischt und zu 5 x 3-mm-Tabletten verformt. Die Tabletten hatten eine Porosität (gemessen über die Wasseraufnahme) von 0,26 ml/g und eine Härte von 4500 N/cm².

Anschließend wurden die Katalysatorformkörper bei einer Temperatur von 450°C und einem Wasserstoffdruck von 250 bar über einen Zeitraum von 20 Stunden reduziert. Nach Abkühlen auf Raumtemperatur erfolgte eine oberflächliche Passivierung mit einem N₂/O₂-Gemisch. Es wurde darauf geachtet, daß im Verlaufe der Passivierung 60°C nicht überschritten wurden.

### Hydrierung von Acetylenalkoholen

### Beispiel 2

Der in Beispiel 1 erhaltene Katalysator wurde in einen Hydrierreaktor eingefüllt. An 8 Volumenteilen des Katalysators wurden bei einer Temperatur von 150°C und einem Wasserstoffdruck von 250 bar 5 Gew.-Teile/h einer 50 %igen wässrigen Lösung von But-2-in-1,4-diol mit 2500 Normvolumenteilen Wasserstoff hydriert. Zur Temperaturführung im Reaktor und zur Gewährleistung der erforderlichen Flüssigkeitsverteilung über das Katalysatorbett wurde der Butindiolzulauf mit 50 Volumenteilen/h Reaktoraustrag verdünnt. Die Hydrierwärme wurde über einen Wärmetauscher im Flüssigkeitskreislauf abgeführt. Nach Abtrennung der Gasphase von der Flüssigphase wurde das überschüssige Gas nach Ergänzung des verbrauchten Anteiles durch Frischwasserstoff zum Reaktoreingang zurückgeführt.

Der Umsatz des But-2-in-1,4-diol erfolgte nahezu vollständig. Nebenprodukte der Hydrierung (jeweils wasserfrei gerechnet) waren u.a.:
- Butanol: < 5 %
- 2-Methylbutan-1,4-diol: < 0,2 %
- But-2-in-1,4-diol: < 0,1 %
- (2-(4-Hydroxi)-butoxi)-oxalen: < 0,3 %
- 4-Hydroxibutyraldehyd: < 0,5 %
- gamma-Butyrolacton: < 0,5 %

Der anfallende Produktaustrag enthielt mindestens 94 % Butan-1,4-diol (berechnet wasserfrei) und wurde destillativ zu Butan-1,4-diol rein aufgearbeitet.

Die Butindiol-Durchsätze aus Beispiel 2 liegen um den Faktor zwei bis drei höher als beim Vergleichsversuch unter Verwendung des in der EP-A-18 569 beschriebenen Katalysators.

### Beispiel 3

Die in Beispiel 2 beschriebene Hydrierung wurde über einen längeren Zeitraum fortgesetzt. Nach einer Betriebszeit von ca. 2 Monaten wurde eine Abnahme der Hydrierleistung des Katalysators beobachtet, welche hauptsächlich durch Ablagerungen anorganischer Bestandteile aus der eingesetzten technischen Butindiollösung verursacht wurde. Das Katalysatorbett wurde 24 Stunden mit 30°C warmem Wasser gespült. Nach dieser Behandlung wies der Katalysator wieder annähernd seine ursprüngliche Aktivität auf. Das Spülwasser enthielt neben organischen Bestandteilen (< 5 Gew.-%) hauptsächlich Silizium (0,1 Gew.-%) und Natrium (0,03 Gew.-%).

## Patentansprüche

1. Verfahren zur Herstellung gesättigter Alkohole durch katalytische Hydrierung von Acetylenalkoholen bei Temperaturen von 50 bis 200°C und Drücken von 30 bis 320 bar, dadurch gekennzeichnet, daß man einen Katalysator verwendet, der einen Gehalt von 20 bis 85 Gew.-% Nickeloxid, 0 bis 30 Gew.-% Kupferoxid, 1 bis 30 Gew.-% Zirkoniumdioxid, 1 bis 30 Gew.-% Siliziumdioxid und 1 bis 30 Gew.-% Aluminiumoxid aufweist, wobei sich die Anteile jeweils auf den oxidischen, nicht reduzierten Katalysator beziehen.

2. Verfahren nach Anspruch 1, dadurch gekennzeichnet, daß man Butan-1,4-diol aus But-2-in-1,4-diol herstellt.

3. Verfahren nach Anspruch 1, dadurch gekennzeichnet, daß man den Katalysator nach Abfall der Aktivität durch eine Behandlung mit Wasser bei Temperaturen von 20 bis 200°C und Drücken von 100 bis 200 bar reaktiviert.

## Claims

1. A process for the preparation of saturated alcohols by catalyst hydrogenation of acetylene alcohols at from 50 to 200°C and at from 30 to 320 bar, which comprises using a catalyst containing from 20 to 85 % by weight of nickel oxide, from 0 to 30 % by weight of copper oxide, from 1 to 30 % by weight of zirconium dioxide, from 1 to 30 % by weight of silicon dioxide and from 1 to 30 % by weight of aluminum oxide, where each of the proportions is based on the oxidic, nonreduced catalyst.

2. A process as claimed in claim 1, wherein butane-1,4-diol is prepared from but-2-yne-1,4-diol.

3. A process as claimed in claim 1, wherein, after a drop in activity, the catalyst is reactivated by treatment with water at from 20 to 200°C and at from 100 to 200 bar.

## Revendications

1. Procédé de préparation d'alcools saturés par hydrogénation catalytique d'alcools acétyléniques à des températures de 50 à 200°C et sous des pressions de 30 à 320 bar, caractérisé en ce qu'on utilise un catalyseur qui présente une teneur de 20 à 85% en poids d'oxyde de nickel, de 0 à 30% en poids d'oxyde de cuivre, de 1 à 30% en poids de dioxyde de zirconium, de 1 à 30% en poids de dioxyde de silicium et de 1 à 30% en poids d'oxyde d'aluminium, ces proportions se rapportant chaque fois au catalyseur à l'état oxydé, non réduit.

2. Procédé selon la revendication 1, caractérisé en ce qu'on prépare du butane-1,4-diol à partir de but-2-yne-1,4-diol.

3. Procédé selon la revendication 1, caractérisé en ce qu'après une baisse de l'activité, on réactive le catalyseur par un traitement par de l'eau à des températures de 20 a 200°C et sous des pressions de 100 à 200 bar.
